# FASCICULE DE BREVET EUROPEEN

(11) **EP 0 506 545 B1**
(45) Date de publication et mention de la délivrance du brevet: **30.12.1998**
(21) Numéro de dépôt: 92400792.5
(22) Date de dépôt: 24.03.1992
(51) Int. Cl.: A61K 31/445

(54) **Utilisation de 4-amino-1-(2-pyridyl)pipéridines comme agonistes 5-HT3 pour le traitement et la prophylaxie des dysfonctionnements sérotoninergiques**
Verwendung von 4-Amino-1-(2-Pyridyl)Piperidinen als 5-HT3-Agonisten zur Behandlung und Verhütung von serotoninergen Dysfunktionen
Use of 4-amino-1-(2-pyridyl)piperidines as 5-HT3-agonists for the treatment and prophylaxis of serotoninergic dysfunctions

(30) Priorité: 27.03.1991 FR 9103735; 03.09.1991 FR 9110890; 15.10.1991 EP 91402753
(43) Date de publication de la demande: 30.09.1992
(73) Titulaire: SANOFI, 75013 Paris (FR); SANOFI WINTHROP S.p.A., 20137 Milano (IT)
(72) Inventeur: Le Fur, Gérard, F-95160 Montmorency (FR); Bianchetti, Alberto, I-20122 Milan (IT); Giudice, Antonina, I-20154 Milan (IT); Croct, Tiziano, I-20122 Milan (IT); Soubrie, Philippe, I-34270 Saint Mathieu de Treviers (IT)
(74) Mandataire: Gillard, Marie-Louise

(56) Documents cités:
- EP-A- 21 973
- NEUROCHEM. INT., vol. 16, no. 3, 1990, pages 309-312, Pergamon Press plc, GB; R.W. FULLER et al.: "Neurochemical effects of CM 57227 and CM 57373, two anorectic agents, on brain serotonin neurons in rats"
- DIALOG INFORMATION SERVICES, INC., File 155, Medline, AN=07215162; S. GARATTINI et al.: "Reduction of food intake by manipulation of central serotonin. Current experimental results", & BR. J. PSYCHIATRY SUPPL., DEC. 1989, (8), P 41-51
- STN INTERNATIONAL, KARLSRUHE DE, FILE CA, CHEMICAL ABSTRACTS, vol. 111, no. 3, abrégé no. 17620k, Columbus, Ohio, US; A.W. SCHMIDT et al.: "Antidepressant interactions with 5-hydroxytrytamine3 receptor binding sites", & EUR. J. PHARMACOL., 1989, 163(2-3), 397-8

## Description

La présente invention concerne une nouvelle utilisation en thérapeutique de certaines 4-amino-1-(2-pyridyl)pipéridines.
EP-B-0021973 décrit la classe des 4-amino-1-(2-pyridyl)pipéridines de formule générale (I) 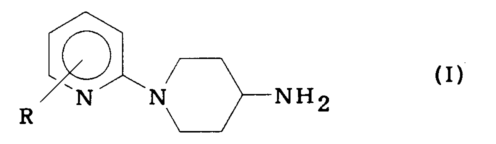 dans laquelle R peut représenter l'hydrogène, un atome d'halogène, un groupe méthyle, trifluorométhyle, (C₁-C₃)alcoxy, trifluorométhoxy, 2,2,2-trifluoroéthoxy, (C₁-C₃)alkylthio, trifluorométhylthio, ou phénoxy ou phénylthio éventuellement substitués par un halogène, un groupe trifluorométhyle, (C₁-C₃)alkyle, (C₁-C₃)alcoxy, (C₁-C₃)alkylthio, ou cyano,
et de leurs sels pharmaceutiquement acceptables, ainsi qu'un procédé pour leur préparation.

Les composés de formule (I) y sont décrits en tant qu'agents anorexigènes essentiellement dépourvus d'effets secondaires majeurs.

Parmi les produits de formule (I) le chlorhydrate de 4-amino-1-(6-chloro-2-pyridyl)pipéridine, décrit dans l'Exemple 1 de EP-B-0021973 et nommé CM 57227, a été soumis aux tests nécessaires pour le passage en clinique humaine, et testé chez l'homme, a été bien toléré jusqu'à une dose de 10 mg.

On a maintenant trouvé que les composés de formule (I) où R représente l'hydrogène, un atome d'halogène, un groupe méthyle, méthylthio, trifluorométhylthio, trifluorométhyle, (C₁-C₃)alcoxy, trifluorométhoxy, 2,2,2-trifluoroéthoxy, ou phénoxy éventuellement substitués par un halogène, un groupe trifluorométhyle, (C₁-C₃)alkyle, (C₁-C₃)alcoxy, (C₁-C₃)alkylthio, ou cyano, ainsi que leurs sels pharmaceutiquement acceptables, décrits dans EP-B-0021973 comme inhibant la capture (dénommée "uptake" en anglais) de la sérotonine, possèdent une activité agoniste sélective vis-à-vis des récepteurs 5-HT₃ au niveau périphérique et central.

Plus particulièrement, on a trouvé que les composés de formule (I) où R a la signification donnée ci-dessus et leurs sels pharmaceutiquement acceptables, ont une affinité pour les sites 5-HT₃ nettement supérieure à celle de la sérotonine et de la 2-méthylsérotonine.

On a aussi trouvé, par des essais de binding ex vixo conduits sur des composés représentatifs de cette classe, que l'affinité pour les sites 5-HT₃ centraux est nettement supérieure à celle pour les sites de capture ("uptake") de la sérotonine.

Un premier objet de la présente invention est donc l'utilisation d'au moins composé de formule (I') 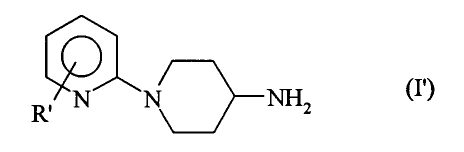 où R' représente l'hydrogène, un atome d'halogène, un groupe méthyle, trifluoro méthyle, (C₁-C₃)alcoxy, trifluorométhoxy, 2,2,2-trifluoroéthoxy, méthylthio, trifluorométhylthio ou phénoxy éventuellement substitué par un halogène, un groupe (C₁-C₃)alkyle, (C₁-C₃)alcoxy, (C₁-C₃)alkylthio, trifluorométhyle ou cyano, ou un de ses sels pharmaceutiquement acceptables, pour la préparation d'un médicament pour le traitement et la prophylaxie des pathologies se rapportant aux troubles du SNC, dans lesquelles une action sérotoninergique provenant de la médiation sélective par les récepteurs 5-HT₃est demandée.

Dans la présente description, le terme "halogène" identifie l'un des quatre halogènes communs, le fluor, le chlore et le brome étant particulièrement préférés.

Les termes "(C₁-C₃)alkyle", (C₁-C₃)alcoxy", et (C₁-C₃)alkylthio" désignent des groupes contenant le résidu d'un hydrocarbure aliphatique saturé contenant 1,2, ou 3 atomes de carbone, à savoir méthyle, éthyle, propyle, et isopropyle.

Les sels pharmaceutiquement acceptables des composés de formule (I') comprennent les sels non toxiques dérivés d'acides minéraux ou organiques salifiant une ou bien les deux fonctions basiques présentes dans les molécules des composés de formule (I') ci-dessus tels que les chlorhydrates, les bromhydrates, les sulfates, les phosphates, les succinates, les tartrates, les fumarates, les maléates, les pamoates, les napsylates, les mesylates, les tosylates, etc.

Une classe préférée de composés de formule (I') comprend les composés (I') où R' est en position 6-du radical 2-pyridyle.

Une classe encore plus avantageuse comprend les composés de formule (I') où R' est l'hydrogène, un atome d'halogène, un groupe méthyle ou un groupe (C₁-C₃)alcoxy en position 6.

Une autre classe préférée de composés de formule (I') comprend les composés (I') où R' est un atome d'halogène en position 3.

La 4-amino-1-(6-chloro-2-pyridyl)pipéridine et ses sels pharmaceutiquement acceptables, notamment le chlorhydrate, la 4-amino-1-(6-bromo-2-pyridyl)pipéridine et ses sels pharmaceutiquement acceptables, notamment le chlorydrate, la 4-amino-1-(6-méthyl-2-pyridyl)pipéridine et ses sels pharmaceutiquement acceptables, notamment le dichlorhydrate, et la 4-amino-1-(6-méthoxy-2-pyridyl)pipéridine et ses sels pharmaceutiquement acceptables, notamment son chlorhydrate, sont les composés particulièrement préférés.

Les composés de formule (I') sont aisement préparés selon la méthode générale décrite dans EP-B-0021973, en faisant réagir la 2-halopyridine correspondante de formule (II) : 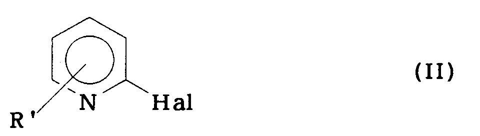 dans laquelle R' a la signification donnée ci-dessus et Hal représente un atome d'halogène, avec une 4-aminopipéridine bloquée de formule (III) 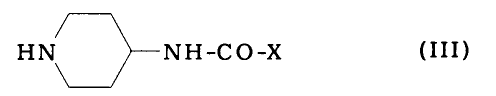 dans laquelle X représente un atome d'hydrogène, ou un groupe (C₁-C₄)alkyle, et en hydrolysant ensuite la 4-amino-1-(2-pyridyl)pipéridine N-acylée ainsi obtenue de formule (IV) 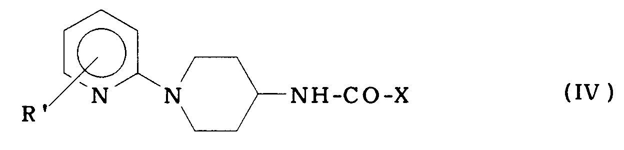 dans des conditions acides ou basiques.

Une description plus détaillée de la méthode générale de préparation des composés (I') ainsi que quelques exemples spécifiques sont contenus dans le brevet européen précité EP-B-0021973.

L'affinité des composés de formule (I') aux récepteurs 5-HT₃ a été évaluée d'abord par un test de binding in vitro en utilisant les sites de liaison 5-HT₃ présents dans le cortex cérébral du rat (cfr. G.J. Kilpatrick, B.J. Jones et M.B. Tyers. Identification and distribution of 5-HT₃ receptors in rat brain using radioligand binding. *Nature 1987; **330**: 746-8*) et comme ligand marqué le [³H] BRL 43694 (granisetron), antagoniste puissant et spécifique des récepteurs 5-HT₃. La puissance des composés de formule (I') dans le déplacement du [³H] BRL 43694 a été comparée à celle de la sérotonine et à celle d'autres agonistes 5-HT₃ (2-méthyl-sérotonine et m-chlorophénylbiguanide), ainsi qu'à celle de l'antagoniste IC 205930.

Pour la préparation des membranes cérébrales, des rats mâles pesant environ 200 g et tués par décapitation, ont été utilisés. La préparation des membranes et le test de liaison ont été effectués selon la méthode reportée par Nelson et Thomas (D.R. Nelson et D.R. Thomas. [³H] BRL 43694 (granisetron), a specific ligand for 5-HT₃ binding sites in rat brain cortical membranes. *Biochem. Pharmacol. 1989*; ***38:** 1963-5*). En résumé, les cortex provenant de 4 animaux ont été homogénéisés dans 20 volumes de HEPES et maintenu à 4°C jusqu'au moment de l'utilisation.

Les membranes ainsi préparées contenaient en moyenne 7,42 (± 0,11) mg de protéines/ml, calculées selon la méthode de Lowry (O.H.Lowry, N.J. Rosenbrough, A.L Fan et R.J. Randall. Protein measurement with the Folin phenol reagent. *J. Biol. Chem. 1951; **193:** 265-75*), en utilisant la sérumalbumine bovine pour la détermination de la courbe de référence.

Pour les expériences de déplacement, 500 µl de la suspension de membrane a été incubée, en présence de [³H] BRL 43694 (activité spécifique 61 Ci/mmol) 0,5 nM et de différentes concentrations de composés non-marqués pendant 30 min. à 25°C, dans un volume final de 1 ml. L'incubation a été bloquée par filtration rapide au travers des filtres Whatman GF/B suivie par 2 lavages avec 10 ml de HEPES 50 mM, pH 7,5, à 4°C. La liaison aspécifique a été déterminée dans des échantillons contenant un excès de IC 205930 (1 µM).

Des études préliminaires de saturation et de cinétique ont été effectuées pour la mise au point du test. Dans l'étude de saturation, 9 concentrations en triplé de [³H] BRL 43694 (de 0,05 à 2 nM) ont été utilisées en déterminant, pour chaque concentration de radioligand, la liaison aspécifique (en présence de ICS 205930, 1 µM).

La cinétique d'association et de dissociation du [³H] BRL 43694 aux sites 5-HT₃ a été suivie pendant 45 minutes, respectivement en absence et en présence de ICS 205930, 1 µM).

Les résultats ont été calculés avec des méthodes d'ajustement ("fitting") non-linéaires : "Accufit saturation", pour les études de saturation, (H.A. Feldman. Mathematical theory of complex ligand-binding systems at equilibrium: some methods of parameter fitting. *Analyt. Biochem. 1972; **48:** 317-38*) et "Accufit competition", pour les études de déplacement, (H.A. Feldman, D. Rodbard et D. Levine. Mathematical theory of cross reactive radioimmunoassay and ligand-binding systems at equilibria. *Analyt. Biochem. 1972; **45**: 530-56*).

L'analyse de Scatchard effectuée sur le [³H] BRL 43694 révèle un site de liaison saturable à haute affinité (K_{d} = 0,46 ± 0,01 nM; Bₘₐₓ = 7,54 ± 0,07 mol/mg de protéines ou 1,12 fmol/mg de tissu frais). Le K_{d} obtenu à partir des courbes d'association et de dissociation, égal à 0,5, est en accord avec celui obtenu à partir de l'analyse de Scatchard.

La concentration de 0,5 nM de [³H] BRL 43694 a donc été utilisée dans les études de compétition afin d'obtenir les affinités de différents composés (Kᵢ).

Dans ce test, les composés de formule (I′) se sont avérés beaucoup (en général 10-20 fois) plus puissants que la sérotonine et la 2-méthyl-sérotonine dans le déplacement du [³H] BRL 43694.

A titre d'exemple, le composé de formule (I′) où R′ est un atome de chlore en position 6 (le CM 57227) a montré une Kᵢ de 193 nM, alors que la sérotonine et la 2-méthyl-sérotonine ont des Kᵢ de 1195 et 1115 nM respectivement.

Cette affinité pour les récepteurs 5-HT₃ est tout à fait sélective. En particulier, on a évalué la capacité du même composé (CM 57227) à déplacer in vitro une série de ligands marqués de différents sites de liaison et, il apparaît clairement des résultats obtenus que ce produit ne possède aucune affinité Kᵢ > 10.000 nM) pour les récepteurs 5-HT_{1A} et 5-HT_{1B}, pour le site d'uptake de la sérotonine (déplacement de la [³H] paroxetine), pour les sites adrénergiques α₁, α₁, β₁ et pour les sites dopaminergiques D₁ et D₂.

On a ensuite confirmé que les composés de formule (I') où R' a la signification donnée ci-dessus pénètrent au niveau du SNC en passant à travers la barrière hématoencéphalique.

Dans ce but, on a conduit des essais ex vivo chez la souris en évaluant l'inhibition de la liaison ("binding") spécifique du [³H] BRL 43694 aux membranes cérébrales du cortex, 30 minutes après l'administration intrapéritonéale (i.p.) de doses croissantes de composés représentatifs de formule (I′).

Dans ces essais, dans lesquels on a suivi la méthodologie décrite par Wood et Piper (J. Psychopharmacol. 1990, **4**(4), 290), on a calculé les valeurs de DI₅₀ (dose inhibant de 50% la liaison du [³H] BRL 43694) obtenues à des dilutions différentes et on a extrapolé la valeur de DI₅₀ à une dilution nulle.

Les composés de formule (I') qui ont été soumis à ce test ont montré une DI₅₀ (mg/kg i.p) comparable à celle du composé GR 38032 F, un antagoniste 5-HT₃ sélectif connu, utilisé comme produit de référence.

En particulier, le CM 57227 a montré une DI₅₀ i.p., extrapolée à dilution nulle, de 1 mg/kg et, en répétant l'essai mais en administrant le composé par voie orale, une DI₅₀ p.o., extrapolée à la dilution nulle, du même ordre de grandeur.

La DI₅₀ vis-à-vis du [³H] BRL 43694 du CM 57227 est nettement inférieure à la DI₅₀ vis-à-vis de la [³H] BRL paroxetine, marqueur des sites de capture ("uptake") de la sérotonine, évaluée dans les mêmes conditions expérimentales.

Le CM 57227 a été aussi étudié dans le test appelé du "turning" conduit selon la méthodologie décrite par P. Worms et al. dans *Life Sciences, 1986, 39 2199-2208*.

Comme pour la 2-méthyl-sérotonine, le chlorhydrate de la 4-amino-1-(6-chloro-2-pyridyl)pipéridine, injecté dans le striatum de souris, provoque des rotations ("turning") inhibées par les antagonistes 5-HT₃. Injecté par voie i.p., ce composé s'oppose à l'antagonisme exercé par l'ondansetron (1 mg/kg i.p.) vis-à-vis du "turning" induit par injection intra-striatale de 2-méthyl-sérotonine avec une DE₅₀ de 0,25 mg/kg, dose tout-à-fait compatible avec le donné ex vivo ci-dessus.

L'activité agoniste sélective des récepteurs 5-HT₃ des composés de formule (I') a été confirmée in vivo dans le test de Bezold-Jarish. Dans ce test, l'administration par voie intraveineuse des composés de formule (I') provoque, chez le rat anesthésié, une diminution fugace de la fréquence cardiaque (effet Bezold-Jarish). L'intensité de l'effet varie selon la dose et est comparable à celle obtenue en administrant de la sérotonine ou de la 2-méthyl-sérotonine.

Cet effet est inhibé par les antagonistes sélectifs des récepteurs 5-HT₃ (par exemple ICS 205930 et zacopride), tandis qu'il n'est pas inhibé par les antagonistes des recepteurs D de la sérotonine (par exemple méthysergide).

Plus particulièrement, l'effet Bezold-Jarish provoqué par les composés de formule (I') a été évalué en utilisant des rats Sprague-Dawley d'un poids compris entre 200 et 300 g, anesthésiés par une dose de 1,25 g/kg intrapéritonéale d'uréthane. La pression artérielle est enregistrée au niveau de l'artère carotide et la fréquence cardiaque évaluée par la fréquence des pulsations à l'aide d'un cardiotachymètre. Un cathéter est placé dans la veine jugulaire pour l'administration des substances.

Différentes doses des composés à tester sont administrées par voie intraveineuse dans un volume de 0,5 ml/kg, et la DI₅₀, c'est-à-dire la dose qui inhibe de 50% la fréquence cardiaque chez les animaux traités, est calculée.

Dans ce test les composés de formule (I') où R' est tel que défini ci-dessus ont montré une DI₅₀ comparable ou inférieure à celle de la sérotonine et à celle de la 2-méthyl-sérotonine.

A titre d'exemple, la DI₅₀ i.v. du CM 57227 est 8,29 µg/kg alors que celle de la sérotonine et de la 2-méthyl-sérotonine est 13,61 et 5,00 µg/kg respectivement.

Les composés de formule (I') où R' a la signification donnée ci-dessus ont montré aussi une activité procinétique intestinale liée à leur action 5-HT₃ agoniste.

Cette activité a été mise en évidence par un test au cours duquel on étudie l'élimination fécale chez le rat.

Selon ce test, des rats mâles pesant entre 220 et 250 g sont laissés 3-4 heures, sans nourriture mais avec libre accès à l'eau dans des cages individuelles. Les composés de formule (I') à l'étude sont administrés par voie sous-cutanée. Dans le schéma de traitement on utilise 8 animaux par groupe.

Après le traitement, l'ampoule rectale est vidée manuellement des selles éventuellement présentes et les animaux sont remis dans leurs cages respectives.

90 minutes après le traitement, les boulettes sont ramassées, comptées et pesées pour la détermination du poids frais. Elles sont ensuite placées dans une étuve et séchées pendant 10 h à 40°C pour la détermination du poids sec.

Le paramètre utilisé pour le calcul de l'activité est le poids sec en grammes des selles réunies cumulativament pendant les 90 minutes qui suivent l'administration par voie sous-cutanée du produit.

Chez les témoins qui reçoivent seulement le véhicule, on n'a presque aucune élimination fécale dans la même période.

Chez les animaux traités avec des doses élevées, on peut avoir une élimination maximale variable de 12-16 boulettes de selles ayant un poids sec d'environ 1,4-1,8 g.

Au-dessus de ces valeurs d'élimination totale, on a l'apparition d'une nette diarrhée qui empêche une quantification exacte.

De ce test il ressort que les composés de formule (I') ont une bonne activité de stimulation de l'élimination fécale à des doses très faibles.

A titre d'exemple, dans ce test le chlorhdyrate de la 4-amino-1-(6-chloro-2-pyridyl)pipéridine (le composé de formule (I') où R' = 6-Cl), à une dose de 2,5 mg/kg s.c. provoque l'élimination d'environ 1,3 g de fèces.

Cette activité est liée à l'activité 5-HT₃ agoniste étant donné que les 5-HT₃ antagonistes selectifs, comme la zacopride ou l'ICS 205930, l'antagonisent.

Sur la base des propriétés ainsi découvertes, les composés de formule (I') sont indiqués pour le traitement des pathologies se rapportant aux troubles du SNC et en particulier des systèmes sérotoninergiques, provenant de la médiation sélective par les récepteurs 5-HT₃.

Les composés de formule (I') où R peut représenter l'hydrogène, un atome d'halogène, un groupe méthyle, trifluorométhyle, (C₁-C₃)alcoxy, trifluorométhoxy, 2,2,2-trifluoroéthoxy, méthylthio, trifluorométhylthio, ou phénoxy éventuellement substitué par un halogène, un groupe (C₁-C₃)alkyle, (C₁-C₃)alcoxy, (C₁-C₃)alkylthio, trifluorométhyle ou cyano, ainsi que leurs sels pharmaceutiquement acceptables, sont donc des agents psychotropes potentiels très intéressants, pouvant agir par l'intermédiaire des récepteurs 5-HT₃.

Plus particulièrement, les composés sus-dits, par leur mécanisme d'action original, sont notamment utiles pour traiter les troubles dysthymiques et peuvent être également utiles dans des cas d'anxiété ou de troubles psychotiques. Ils peuvent aussi être utiles pour le traitement des troubles de la motricité intestinale et en particulier pour le traitement de la constipation.

En général, ils peuvent être utilisés dans le traitement de toutes pathologies dans lesquelles une action sérotoninomimétique provenant de la médiation sélective par les récepteurs 5-HT₃ peut être bénéfique.

L'invention prévoit donc l'utilisation des composés (I') où R' a la signification donnée ci-dessus pour la préparation de médicaments destinés au traitement et à la prophylaxie des pathologies mentionnées ci-dessus.

En plus, les composés de formule (I') sont peu toxiques, leur toxicité étant bien compatible avec une utilisation en thérapeutique.

Pour l'emploi dans les pathologies susdites, les composés de formule (I') ainsi que leurs sels pharmaceutiquement acceptables, peuvent être convenablement administrés par voie orale, parentérale, sublinguale, rectale ou transdermique, élaborés sous forme de compositions pharmaceutiques.

La quantité de principe actif à administrer par jour dépend de la particularité de l'indication thérapeutique, de la gravité des affections à traiter, ainsi que du poids du malade et de la voie d'administration.

De toute façon la dose globale chez l'homme varie en général entre 0,05 et 100 mg par jour, par exemple de 0,1 à 50 mg, et plus convenablement de 0,5 à 20 mg par jour.

Les compositions pharmaceutiques préparées en utilisant des composés de formule (I') et destinées à combattre les pathologies sus-dites, renferment au moins un produit choisi parmi les composés de formule (I') et leurs sels d'addition pharmaceutiquement acceptables en association avec un véhicule pharmaceutiquement inerte.

Elles peuvent être préparées selon des méthodes usuelles bien connues dans le domaine de la galénique.

Le principe actif peut être incorporé à des excipients habituellement employés dans ces compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les différents agents mouillants, dispersants, ou émulsifiants, les conservateurs, etc.

Pour l'administration par voie orale qui est de toute façon la voie d'administration préférée, des formes pharmaceutiques appropriées comprennent les comprimés, les comprimés retard, les dragées, les gélules, les suspensions, les solutions, ou encore les liposomes.

En ce qui concerne l'administration intraveineuse, sous-cutanée ou intramusculaire, on recourt à des solutions stériles ou stérilisables, tandis qu'on peut réaliser des suppositoires conventionnels, ou des gélules ou des microclystères pour l'administration rectale.

Pour l'administration transdermique on peut utiliser des patches conventionnels préparés selon des techniques bien connues par l'homme du métier.

Les formes unitaires de dosage pour la nouvelle utilisation thérapeutique comprendront en général de 0,05 à 20 mg, allant de préférence de 0,1 à 10 mg, y compris par exemple le dosage de 0,5 à 5 mg (notamment 0,5 - 1 - 1,5 - 2 - 2,5 - 3 - 3,5 - 4 - 4,5 et 5 mg de produit). Ces doses unitaires sont administrées normalement une ou plusieurs fois par jour, de préférence une à trois fois par jour.

Si on le désire, les compositions pharmaceutiques de la présente invention peuvent contenir aussi un ou plusieurs autres médicaments connus et communément utilisés pour les mêmes indications thérapeutiques.

## Revendications

1. Utilisation d'au moins un composé de formule (I') 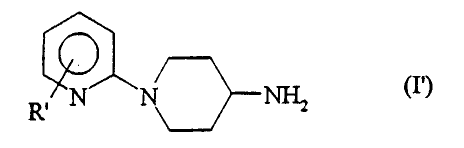 où R' représente l'hydrogène, un atome d'halogène, un groupe méthyle, trifluorométhyle, (C₁-C₃)alcoxy, trifluorométhoxy, 2,2,2-trifluoroéthoxy, méthylthio, trifluorométhylthio ou phénoxy éventuellement substitué par un halogène, un groupe (C₁-C₃)alkyle, (C₁-C₃)alcoxy, (C₁-C₃)alkylthio, trifluorométhyle ou cyano, ou un de ses sels pharmaceutiquement acceptables, pour la préparation d'un médicament pour le traitement et la prophylaxie des pathologies se rapportant aux troubles du SNC, dans lesquelles une action sérotoninergique provenant de la médiation sélective par les récepteurs 5-HT₃ est demandée.

2. Utilisation selon la revendication 1 dans laquelle R' est en position 6- du radical 2-pyridyle.

3. Utilisation selon la revendication 1 dans laquelle R' représente un atome d'hydrogène, un atome d'halogène, un groupe méthyle, ou un groupe (C₁-C₃)alcoxy en position 6-.

4. Utilisation selon la revendication 3 dans laquelle le composé de formule (I') est choisi parmi la 4-amino-1-(6-chloro-2-pyridyl)pipéridine, la 4-amino-1-(6-bromo-2-pyridyl)pipéridine, la 4-amino-1-(6-méthyl-2-pyridyl)pipéridine, la 4-amino-1-(6-méthoxy-2-pyridyl)pipéridine et leurs sels pharmaceutiquement acceptables.

5. Utilisation selon la revendication 4 dans laquelle le composé de formule (I') est la 4-amino-1-(6-chloro-2-pyridyl)pipéridine ou un de ses sels pharmaceutiquement acceptables.

6. Utilisation selon la revendication 1 dans laquelle R' est un atome d'halogène en position 3- du radical 2-pyridyle.

7. Utilisation selon l'une quelconque des revendications 1 à 6 caractérisée en ce que le médicament est psychotrope.

8. Utilisation selon la revendication 7 caractérisée en ce que le médicament est destiné à combattre les troubles dysthymiques, l'anxiété ou les troubles psychotiques.

9. Utilisation selon l'une quelconque des revendications 1 à 6 caractérisée en ce que le médicament est destiné au traitement des troubles de la motricité intestinale.

10. Utilisation selon la revendication 9 caractérisée en ce que le médicament est destiné au traitement de la constipation.

## Claims

1. Use of at last one compound of formula (I') 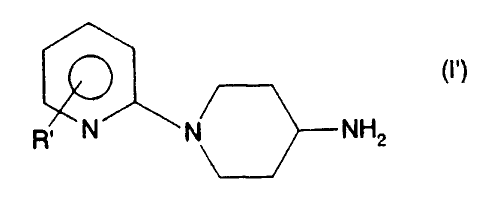 where R' represents hydrogen, a halogen atom, a methyl, trifluoromethyl, (C₁-C₃)alkoxy, trifluoromethoxy, 2,2,2-trifluoroethoxy, methylthio, trifluoromethylthio, or phenoxy group optionally substituted with a halogen atom, a (C₁-C₃)alkyl, (C₁-C₃)alkoxy, (C₁-C₃)alkylthio, trifluoromethyl or cyano group, or a pharmaceutically acceptable salt thereof, for the preparation of a medicament for the treatment and the prophylaxis of pathologies related to disorders of the CNS in which a serotoninergic action selectively mediated by 5-HT₃ receptors is required.

2. Use according to claim 1, in which R' is at position 6- of the 2-pyridyl radical.

3. Use according to claim 1, in which R' represents a hydrogen atom, a halogen atom, a methyl or (C₁-C₃)alkoxy group at position 6-.

4. Use according to claim 3, in which the compound of formula (I') is selected from the group consisting of 4-amino-1-(6-chloro-2-pyridyl)piperidine, 4-amino-1-(6-bromo-2-pyridyl)piperidine, 4-amino-1-(6-methyl-2-pyridyl)piperidine, 4-amino-1-(6-methoxy-2-pyridyl)piperidine and the pharmaceutically acceptable salts thereof.

5. Use according to claim 4, in which the compound of formula (I') is 4-amino-1-(6-chloro-2-pyridyl)pipendine or a pharmaceutically acceptable salt thereof.

6. Use according to claim 1, in which R' is a halogen atom at position 3- of the 2-pyridyl radical.

7. Use according to any one of claims 1 to 6, characterized in that it is a psychotropic medicament.

8. Use according to claim 7, characterized in that the medicament is suitable for the treatment of dysthymic disorders, anxiety or psychotic disorders.

9. Use according to any one of claims 1 to 6, characterized in that the medicament is suitable for the treatment of intestinal motoricity disorders.

10. Use according to claim 9, characterized in that the medicament is suitable for the treatment of constipation.

## Patentansprüche

1. Verwendung mindestens einer Verbindung der Formel (I'), 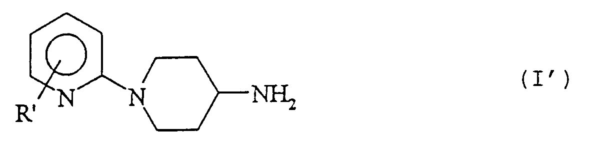 in der R' Wasserstoff, ein Halogenatom, Methyl, Trifluormethyl, C₁₋₃-Alkoxy, Trifluormethoxy, 2,2,2,-Trifluorethoxy, Methylthio, Trifluormethylthio oder Phenoxy, das gegebenenfalls mit einem Halogen, C₁₋₃-Alkyl, C₁₋₃-Alkoxy, C₁₋₃-Alkylthio, Trifluormethyl oder Cyano substituiert ist, bedeutet, oder eines ihrer pharmazeutisch akzeptablen Salze, für die Herstellung eines Arzneimittels zur Behandlung von und der Vorbeugung vor Krankheiten, die mit Störungen des Zentralnervensystems in Zusammenhang stehen, bei denen eine serotoninerge Wirkung, die von der selektiven Vermittlung durch 5-HT₃-Rezeptoren herrührt, erforderlich ist.

2. Verwendung nach Anspruch 1, wobei R' in 6-Stellung des 2-Pyridyl-Rests angeordnet ist.

3. Verwendung nach Anspruch 1, wobei R' ein Wasserstoffatom, ein Halogenatom, eine Methylgruppe oder eine C₁₋₃-Alkoxygruppe in 6-Stellung darstellt.

4. Verwendung nach Anspruch 3, wobei die Verbindung der Formel (I') unter 4-Amino-1-(6-chlor-2-pyridyl)-piperidin, 4-Amino-1-(6-brom-2-pyridyl)-piperidin, 4-Amino-1-(6-methyl-2-pyridyl)-piperidin, 4-Amino-1-(6-methoxy-2-pyridyl)-piperidin und deren pharmazeutisch akzeptablen Salzen ausgewählt wird.

5. Verwendung nach Anspruch 4, wobei die Verbindung der Formel (I') das 4-Amino-1-(6-chlor-2-pyridyl)-piperidin oder eines seiner pharmazeutisch akzeptablen Salze ist.

6. Verwendung nach Anspruch 1, wobei R' ein Halogenatom in 3-Stellung des 2-Pyridyl-Rests ist.

7. Verwendung nach einem der Ansprüche 1 bis 6, dadurch gekennzeichnet, daß das Arzneimittel psychotrop ist.

8. Verwendung nach Anspruch 7, dadurch gekennzeichnet, daß das Arzneimittel für die Bekämpfung von dysthymischen Störungen, Angst oder psychotischen Störungen vorgesehen ist.

9. Verwendung nach einem der Ansprüche 1 bis 6, dadurch gekennzeichnet, daß das Arzneimittel für die Behandlung von Störungen der Darmmotilitat vorgesehen ist.

10. Verwendung nach Anspruch 9, dadurch gekennzeichnet, daß das Arzneimittel für die Behandlung der Verstopfung vorgesehen ist.
